Europäisches Patentamt

(19) European Patent Office

Office européen des brevets

(11) Publication number: **0 458 652 A1**

(12) **EUROPEAN PATENT APPLICATION**

(21) Application number: **91304746.0**

(22) Date of filing: **24.05.91**

(51) Int. Cl.⁵: **C07K 1/14, C07K 3/22**

(30) Priority: **24.05.90 GB 9011661**

(43) Date of publication of application:
**27.11.91 Bulletin 91/48**

(84) Designated Contracting States:
**AT BE CH DE DK ES FR GB GR IT LI LU NL SE**

(71) Applicant: **CELLTECH LIMITED**
**216 Bath Road**
**Slough Berkshire SL1 4EN (GB)**

(72) Inventor: **Cutler, Paul**
**18 Hedley Road**
**Herts., AL1 5JW (GB)**
Inventor: **Brady, David John**
**39 Stoney Grove Cameron Road**
**Chesham, Bucks. (GB)**
Inventor: **Bonnerjea, Julian Roy**
**23 Bassano Street, East Dulwich**
**London, SE22 (GB)**

(74) Representative: **Hallybone, Huw George et al**
**CARPMAELS AND RANSFORD 43 Bloomsbury**
**Square**
**London WC1A 2RA (GB)**

(54) A process for the purification of proteins.

(57) A process is described for separating mixtures of proteins and deoxyribonucleic acids (DNA). The process utilises immobilised polyamines such as polyethylemeimine with which a protein containing DNA solution can be mixed in the presence of an inert inorganic salt, optionally in the presence of a zwitterionic buffer. Under these conditions, protein may be subsequently recovered from the immobilised polyamine to contain less than 1pg DNA per milligram of protein. The process is particularly useful for removing DNA from antibodies.

EP 0 458 652 A1

Jouve, 18, rue Saint-Denis, 75001 PARIS

## Field of the Invention

This invention relates to a process for the purification of proteins, in particular to a process for separating proteins and deoxyribonucleic acids.

## Background to the Invention

The widespread use of proteins in biological research, and for diagnosis and therapy, has created a demand for preparations of these substances that are free of contaminants that would otherwise interfere with the intended use of the protein and/or, when the use is for therapy, cause unwanted side-effects in the subject undergoing treatment. Particularly undesirable contaminants include deoxyribonucleic acids (DNAs) which are especially found associated with proteins produced using cell culture techniques. Before such proteins can be reliably and safely used in research and medicine, it is essential that DNA contamination is reduced to an acceptably low level.

Many traditional methods for the purification of proteins from cell culture have been used to separate DNA and protein including the use of centrifugation, ultrafiltration, and gel exclusion, affinity and standard ion exchange, e.g. DEAE, chromatography . In our hands, however, none of these techniques has proved to be suitable for the removal of DNAs from protein solutions such that a good yield of protein with an acceptable low level of DNA contamination [i.e. less than 1pg DNA per mg protein] is obtained.

We have now found that it is possible to achieve a good separation of proteins and DNAs, even when the latter are in low concentration, by using a polyamine ion exchanger, particularly a polyethyleneinine ion exchanger, and we have used this discovery to develop a process for obtaining protein solutions in good yield and substantially free of DNA contamination.

## Summary of the Invention

Thus according to one aspect of the present invention we provide a process for the preparation of a protein solution substantially free of DNA which comprises the steps of (1) contacting a protein solution containing DNA with a medium comprising a hydrophilic matrix to which are attached polyamine groups in the presence of an inert inorganic salt, (2) washing the medium to effect separation of said protein and DNA and (3) recovering a protein solution substantially free of DNA from the medium.

In this aspect of the invention the term "protein solution substantially free of DNA" is intended to mean any protein solution containing up to and including 1pg of DNA per milligram of protein.

In the process according to the invention, the protein starting material may be for example an aqueous solution of a naturally occurring or artificially obtained protein or polypeptide or a derivative thereof. Artificially obtained proteins or polypeptides and derivatives thereof include those obtained by recombinant DNA means or by chemical synthesis. Typical starting materials include for example solutions of immunoglobulins, such as polyclonal, monoclonal or recombinant antibodies [for example chimeric or CDR-grafted antibodies] and fragments thereof [e.g. $F(ab)_2$, Fab or Fv fragment], and single chain antibodies; hormones, such as erythropoietin and growth hormone, e.g. human growth hormone; lymphokines such as interferon or tumour necrosis factor; interleukins such as interleukin 1 or interleukin 2; industrially and therapeutically useful enzymes such as tissue plasminogen activator; and enzyme inhibitors such as tissue inhibitor of metalloproteinases. The process according to the invention is particularly suitable for use with immunoglobulin starting solutions.

The medium for use in the process according to the invention may comprise a hydrophilic insoluble support such as a cellulose, cross-linked agarose, cross-linked dextran, or synthetic polymer e.g. polyacrylamide or silica or a derivative thereof, to which are attached optionally cross-linked polyamine groups. Polyamine groups include lysine, spermine or, especially, polyethyleneimine groups. A preferred medium comprises a hydrophilic insoluble support, particularly a silica matrix or a derivative thereof to which are attached optionally cross-linked polyethyleneimine groups. A particularly preferred medium is that available from J.T. Baker Inc. (Phillipsburg, NJ) and sold as "Bakerbond Wide-Pore PEI".

The inert inorganic salt for use in the process according to the invention may in general be any inorganic salt which is soluble in an aqueous medium. Thus, for example, the salt may be a monovalent or divalent inorganic salt, such as an alkali or alkaline earth metal or ammonium salt, for example a halide e.g. chloride, or sulphate. Particular salts include for example sodium or potassium chloride, or ammonium sulphate.

The exact concentration of the salt will vary depending on the nature of the protein solution, the medium, and other factors such as pH, and may be easily determined in accordance with conventional practice using preliminary small-scale tests. In general, however, the salt may be present in a concentration of around 0.1M up to the solubility limit of the salt, for example from around 0.1M up to around 4.0M, e.g. 0.5M to 1.0M.

The process according to the invention may be effected using either a batch or, preferably, column or cartridge separation technique, in accordance with conventional practice. Thus, in general, the protein starting solution will be added to the medium such that any DNA contaminant will bind to the medium. This may be achieved by adjusting the ionic strength of the starting solution by use of the inert inorganic salt to a suitable pre-determined value and pre-equilibrating the medium with any suitable buffer of ionic strength comparable to that of the starting solution.

The protein solution for addition to the medium may be prepared in any suitable way. Thus, for example a dried protein preparation may be dissolved in a suitable buffer containing an inert inorganic salt as just described at a concentration to yield an appropriate ionic strength, for example an ionic strength equivalent to the ionic strength of a solution containing sodium chloride at a concentration in the range 0.1M to 5.0M. When the protein starting material is already in solution, the ionic strength of the solution may be adjusted to the desired value (as just described), for example by addition of a salt as just described or alternatively by buffer exchange using for example dialysis or gel filtration chromatography. Suitable buffers include inorganic, e.g. phosphate, buffers (for example at a concentration in the range 10-100mM) and, in particular, zwitterionic organic buffers such as amino acid buffers.

Zwitterionic organic buffers have proved to be particularly useful for clearing DNA in the process according to the invention, and according to a further aspect of the invention we provide a process for the preparation of a protein solution substantially free of DNA which comprises the steps of (1) contacting a protein solution containing DNA with a medium comprising a hydrophilic matrix to which are attached polyamine groups in the presence of an inert organic salt and a zwitterionic organic buffer, (2) washing the medium to effect separation of said protein and DNA and (3) recovering a protein solution substantially free of DNA from the medium.

A hydrophilic matrix to which are attached polyethyleneimine groups is particularly useful according to this aspect of the invention.

The zwitterionic organic buffer for use in this aspect of the invention may be for example an amino acid buffer, such as a histidine buffer or an aminosulphonic acid buffer such as a morpholino ethane sulphonic acid buffer. The zwitterionic buffer may be at any suitable concentration, for example in the range 10-100mM e.g. around 50mM.

The pH of the protein starting solution may be varied within a wide range depending on the nature of the protein, and the exact pH for optimum separation of protein and DNA may be determined beforehand using appropriate small-scale tests. In general the pH may be varied from around pH5.0 to around pH10 for example pH5.0 to pH10, such as pH6.0-pH8.0.

Once the protein solution has been added to the medium, washing of the medium with an elution buffer of suitable ionic strength will then separate the DNA and protein. Thus, for example when the protein does not bind to the medium in the initial buffer and at the salt concentration selected the elution buffer may be for example at an ionic strength comparable to that of the protein starting solution and elution may be continuous with application. When the protein does bind to the medium, the elution buffer may be at an ionic strength higher than the initial buffer, to effect removal of the protein and separation from any DNA.

When a batch separation technique is in use the protein may be recovered from the medium by filtration or centrifugation When a column, cartridge or filter separation technique is used, the protein may be recovered by collection of appropriate eluted fractions as the column is washed.

If desired, or as necessary, the process according to the invention may be repeated to obtain a protein solution with the required DNA level.

During the process according to the invention, the presence of protein in any solution may be determined using conventional protein detection techniques, for example ultraviolet absorption spectroscopy. Similarly, the presence of DNA in any solution may be determined using standard assay procedures, such as a molecular hybridisation assay.

Description of Specific Embodiments

The following Examples illustrate the invention. Protein and DNA were assayed using the techniques just described and compared with appropriate standards.

Example 1

(a) Use of Bakerbond Wide Pore Polyethyleneimine Matrix (WP-PEI) to remove DNA from Monoclonal Antibody

Column Preparation

1. A column was prepared using the minimum of 7.5ml WP-PEI to 1g of monoclonal IgG antibody that had previously been purified by protein A affinity and DEAE anion-exchange chromatography.

2. The matrix was weighed out (1g of dry weight is equal to 3ml of wet matrix) and equilibration buffer, (50mM histidine/0.5M NaCl pH6.0), was added to give a 50:50 v/v slurry.

3. The slurry was poured into an appropriate column and washed with 1 column volume of 6M guanidine hydrochloride.

4. The column was then equilibrated with around 10 column volumes of equilibration buffer (as above).

Operation

1. The above antibody (containing around 100pg murine DNA per mg antibody) was buffer-exchanged into the equilibration buffer at a concentration of between 15-10mg IgG ml⁻¹.

2. The antibody was loaded onto the column at 50cm hr⁻¹. Under these conditions the antibody did not bind to the column and was collected in the flow through.

3. After loading the antibody the column was washed with 1 volume of equilibration buffer to effect complete elution.

4. The recovered antibody contained less than 1pg DNA per mg antibody.

(b) The above experiment was repeated using the following matrices:

    polyethyleneimine - cellulose (Sigma)
    lysine - sepharose (Pharmacia)
    spermine - agarose (Sigma)

and a 100mM phosphate, pH7.0 equilibration buffer containing 0.1 NaCl.

In each instance the antibody starting material (containing around 100pg murine DNA per mg antibody) did not bind to the matrix and was collected in the flow through. The recovered antibody contained less than 1pg DNA per mg antibody.

## Example 2

The procedure of Example 1(a) was repeated, except that the concentration of the antibody starting solution was varied. Analysis of the recovered antibody showed that the concentration of DNA (less than 1pg DNA per mg antibody) was independent of the initial antibody concentration [2.5; 5.0; 10.0; 15.0; mg ml⁻¹] used.

## Claims

1. A process for the preparation of a protein solution substantially free of DNA which comprises the steps of (1) contacting a protein solution containing DNA with a medium comprising a hydrophilic matrix to which are attached polyamine groups in the presence of an inert inorganic salt, (2) washing the medium to effect separation of said protein and DNA and (3) recovering a protein solution substantially free of DNA from the medium.

2. A process according to Claim 1 wherein the protein is an immunoglobulin.

3. A process according to Claims 1 or 2 wherein the polyamine is polyethyleneimine

4. A process according to Claims 1 to 3 wherein step (1) is performed in the presence of a zwitterionic organic buffer.

5. A process according to Claim 4 wherein the zwitterionic organic buffer is an amino acid buffer.

6. A process according to Claim 5 wherein the amino acid buffer is a histidine buffer.

7. A process according to any of the preceding claims wherein the pH of the protein solution in step (1) is in the range around pH5.0 to around pH10.0.

8. A process according to Claim 7 wherein the pH is in the range pH6.0 to pH8.0.

9. A process according to any of the preceding claims wherein the inert inorganic salt is at a concentration in the range around 0.1M to around 4.0M.

10. A process according to Claim 9 wherein the inert inorganic salt is at a concentration in the range of 0.5M to 1.0M.

European Patent Office

**EUROPEAN SEARCH REPORT**

Application Number

EP 91 30 4746

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | PROTEIN PURIFICATION, UCLA SYMPOSIA ON MOLECULAR AND NEW SERIES, vol. 68: "Cellular Biology", 1987, pages 75-97, Alan R. Liss, Inc., New York, US; J. JENDRISAK: "The use of polyethyleneimine in protein purification" * The whole document * | 1,3,7-10 | C 07 K 1/14<br>C 07 K 3/22 |
| Y | IDEM | 4-6 | |
| X | BIOCHEMISTRY, vol. 14, no. 21, 1975, pages 4639-4645, Washington, US; J.J. JENDRISAK et al.: "A new method for the large-scale purification of wheat germ DNA-dependent RNA polymerase II" * Page 4639 (introduction); Page 4640, column 1 (buffers); page 4642, column 2 * | 1,3,7-9 | |
| Y | IDEM | 4-6 | **TECHNICAL FIELDS SEARCHED (Int. Cl.5)** |
| Y | DNA, vol. 7, no. 1, 1988, pages 57-62, New York, US; W. MANDECKI et al.: "High-resolution polyacrylamide gel electrophoresis of oligonucleotides using L-histidine buffer" * Pages 57-58 (abstract and introduction); pages 60-62 (discussion) * | 4-6 | C 07 K |

The present search report has been drawn up for all claims

| Place of search | Date of completion of the search | Examiner |
|---|---|---|
| THE HAGUE | 28-08-1991 | KORSNER S.E. |